# EUROPEAN PATENT APPLICATION

(11) **EP 4 442 826 A1**
(43) Date of publication of application: **09.10.2024**
(21) Application number: 23167017.5
(22) Date of filing: 06.04.2023
(51) Int. Cl.: C12N 15/11, C12N 15/85

(54) **SYNTHETIC DNA CONSTRUCT ENCODING TRANSFER RNA**

(71) Applicant: Universität Hamburg, 20148 Hamburg (DE)
(72) Inventor: IGNATOVA, Zoya, 20146 Hamburg (DE); ALBERS-FOMENKO, Suki, 20146 Hamburg (DE); DAVYT, Marcos, 20146 Hamburg (DE); BHARTI, Nikhil, 20146 Hamburg (DE)
(74) Representative: Stüven, Ralf

(57) **Abstract**

The invention relates to a synthetic DNA construct comprising (A) a nucleic acid encoding a transfer RNA and (B) a 5' leader sequence, the 5' leader sequence comprising one or up to three sequence motifs for controlling the expression level of the transfer RNA.

## Description

The invention relates to a synthetic DNA construct encoding tRNA, that can be used, for example, for the delivery of transfer RNA into cells, for example human cells.

Transfer ribonucleic acids (tRNAs) are an essential part of the protein synthesis machinery of living cells as necessary components for translating the nucleotide sequence of a messenger RNA (mRNA) into the amino acid sequence of a protein. Naturally occurring tRNAs comprise an amino acid binding stem being able to covalently bind an amino acid and an anticodon loop containing a base triplet called "anticodon", which can bind non-covalently to a corresponding base triplet called "codon" on an mRNA. A protein is synthesized by assembling the amino acids carried by tRNAs using the codon sequence on the mRNA as a template with the aid of a multi component system comprising, inter alia, the ribosome and several auxiliary enzymes.

Transfer RNAs have recently seen increasing interest in their use as drugs for therapeutic purposes, e.g. as part of a gene therapy for treating conditions associated with nonsense mutations, i.e. mutations changing a sense codon encoding one of the twenty amino acids specified by the genetic code to a chain-terminating codon ("premature termination codon", PTC) in a gene sequence (Ai-Ming Yu, Young Hee Choi and Mei-Juan Tu, RNA Drugs and RNA Targets for Small Molecules: Principles, Progress, and Challenges, Pharmacological Reviews, 2020, 72 (4) 862-898; DOI: 10.1124/pr. 120.019554; Porter, JJ, Heil, CS, Lueck, JD, Therapeutic promise of engineered nonsense suppressor tRNAs, WIREs RNA. 2021; 12:e1641, DOI: 10.1002/wrna.1641). Lueck et al. 2016 (Lueck, J.D., Infield, DT, Mackey, AL, Pope, RM, McCray, PB, Ahern, CA. Engineered tRNA suppression of a CFTR nonsense mutation, bioRxiv 088690; doi: 10.1101/088690), for example, describe a codon-edited tRNA enabling the conversion of an in-frame stop codon in the CFTR gene to the naturally occurring amino acid in order to restore the full-length wild type protein.

Compared to mRNA, tRNA molecules offer significantly higher stability and are on average 10-fold shorter, alleviating the problem of introduction into the target tissue. This has led to attempts to use tRNA in gene therapy in order to prevent the formation of a truncated protein from an mRNA with a premature stop codon and to introduce the correct amino acid instead (see, e.g., Koukuntla, R., 2009, Suppressor tRNA mediated gene therapy, Graduate Theses and Dissertations, 10920, Iowa State University, http://lib.dr.iastate.edu/etd/10920; US 2003/0224479 A1; US 6964859). WO 2017/121863 A1 and WO 2020/208169 A1 describe synthetic tRNA with an extended anticodon loop that can, for example, be used as a suppressor tRNA for genetic diseases associated with a frameshift mutation. WO 2021/113218 A1 discloses engineered tRNA molecules and vectors encoding engineered suppressor tRNA molecules that recognize and readthrough of disease-causing premature stop codons. The use of tRNA in the context of gene therapy, e.g., of diseases associated with the existence of premature termination codons (PTCs), for example, is also described, inter alia, in WO 2020/069194 A1, WO 2021/211762 A2, WO 2021/087401 A1, WO 2021/113218 A1, and US 2020/291401 A1.

The biogenesis of tRNA comprises several processes, including transcription, 5' and 3' ends processing, splicing, post-transcriptional nucleotide modification, CCA addition and aminoacylation. The transcription of tRNA involves binding of the transcription factor TFIIIC to intragenic sequence motifs (promoters), called "A box" and "B box", coding for parts of the D- and T arm, respectively, and the recruitment of the transcription factor TFIIIB to the 5'-upstream region of the tRNA gene, which directs recruitment of RNA polymerase III (pol III) transcribing the tRNA gene (see, for example, Kirchner, S., Ignatova, Z. Emerging roles of tRNA in adaptive translation, signalling dynamics and disease, Nat Rev Genet 16, 98-112 (2015), doi: 10.1038/nrg3861; Schramm L, Hernandez N., Recruitment of RNA polymerase III to its target promoters, Genes Dev. 2002 Oct 15;16(20):2593-620, doi: 10.1101/gad.1018902; Mitra S., Das P., Samadder A., Das S., Betai R., Chakrabarti J., 2015, Eukaryotic tRNAs fingerprint invertebrates vis-à-vis vertebrates, Journal of Biomolecular Structure and Dynamics, doi: 10.1080/07391102.2014.990925; Maraia, R.J., Arimbasseri, A.G., It's Sno'ing on Pol III at nuclear pores. Genome Biol 14, 137, 2013, doi: 10.1186/gb4137; Canella D, Bemasconi D, Gilardi F, LeMartelot G, Migliavacca E, Praz V, Cousin P, Delorenzi M, Hernandez N; CycliX Consortium. A multiplicity of factors contributes to selective RNA polymerase III occupancy of a subset of RNA polymerase III genes in mouse liver. Genome Res. 2012 Apr;22(4):666-80. doi: 10.1101/gr.130286.111; Enserink JM, Chymkowitch P. Cell Cycle-Dependent Transcription: The Cyclin Dependent Kinase Cdk1 Is a Direct Regulator of Basal Transcription Machineries. Int J Mol Sci. 2022 Jan 24;23(3):1293, doi: 10.3390/ijms23031293; Berg MD, Brandl CJ., Transfer RNAs: diversity in form and function, RNA Biol. 2021 Mar; 18(3):316-339, doi: 10.1080/15476286.2020.1809197; Korde A., Extra-Transcriptional Effects of Chromatin Bound RNA Polymerase III Transcription Complexes, 2014. LSU Doctoral Dissertations. 274, https://digitalcommons.lsu.edu/gradschool_dissertations/274, doi: 10.31390/gradschool_dissertations.274; Young LS, Rivier DH, Sprague KU. Sequences far downstream from the classical tRNA promoter elements bind RNA polymerase III transcription factors, Mol Cell Biol. 1991 Mar; 11(3):1382-92, doi: 10.1128/mcb.11.3.1382-1392.1991;). Zhang et al. 2011 (Zhang G, Lukoszek R, Mueller-Roeber B, Ignatova Z. Different sequence signatures in the upstream regions of plant and animal tRNA genes shape distinct modes of regulation. Nucleic Acids Res. 2011 Apr;39(8):3331-9. doi: 10.1093/nar/gkq1257) describe anticodon-dependent sequence motifs within a non-coding 5'-upstream region of tRNA genes that might be involved in the regulation of tRNA transcription.

It is an object of the invention to improve the means and possibilities for gene therapy of diseases using transfer RNA. In particular, it is an object of the invention, to provide improved means for the delivery of suppressor tRNA to living cells, for example human cells.

For solving the problem, the invention provides, in one aspect, a synthetic DNA construct comprising (A) a nucleic acid encoding a transfer RNA and (B) a 5' leader sequence, the 5' leader sequence comprising
a) at least one sequence motif selected from the group consisting of the sequence motifs TGACCTAAGTGTAAAGT, I_{H}, (SEQ ID NO: 1), TGAGATTTCCTTCAGGTT, II_{H}, (SEQ ID NO: 2), TATATAGTTCTGTATGAGACCACTCTTTCCC, III_{H}, (SEQ ID NO: 3), ACCATAAACGTGAAATG, I_{L}, (SEQ ID NO: 4), TCTTTGGATTTGGGAATC, II_{L}, (SEQ ID NO: 5, and TTATAAGTTCTGTATGAGACCACTCTTTCCC, III_{L}, (SEQ ID NO: 6); or
b) one sequence motif selected from the group consisting of the sequence motifs
   VNANANTVHANANNTTNNNATRANATTCNGDGVGNAANATABTNCTVGND, 50nt_{H}, (SEQ ID NO: 7) and
   GCNGGDGGCGNGTTCBBCTGTNVNAGCNTNCGDNGNCGTTTNNCNTCGGT, 50nt_{L}, (SEQ ID NO: 8); or
c) at least one sequence motif selected from the group consisting of the sequence motifs GAAATGCCTT, 10nt_{H1}, (SEQ ID NO: 9), GTGGGAACTA, 10nt_{H2}, (SEQ ID NO: 10), and GTGTTGCTTG, 10nt_{H3}, (SEQ ID NO: 11).

The invention provides a novel synthetic DNA construct comprising a nucleic acid encoding a transfer RNA. The DNA construct can, for example, be configured as a gene delivery vehicle (GDV) for the delivery of transfer RNA, for example suppressor tRNA, into cells, in particular mammalian cells, e.g., human cells. Besides a tRNA gene, the synthetic DNA construct of the invention comprises a 5' leader sequence functionally linked to the nucleic acid encoding the tRNA (tRNA gene), wherein the 5' leader sequence comprises sequence motifs for the binding of the transcription factor TFIIIB. The invention provides sequence motifs having promoter activity that can be included in a 5' leader sequence of a tRNA encoded downstream of the 5' leader sequence. Suitable selection and, as the case may be, combination of the sequence motifs enables control of the transcription of the downstream tRNA gene. The tRNA encoded by the nucleic acid in the construct may, for example, be an engineered tRNA, having, for example, a modified anticodon being able to base-pair with a stop codon on an mRNA, and/or a modified T arm comprising a B box sequence motif. The B box sequence may, for example, be a sequence enhancing the binding of the transcription factor TFIIIC to the tRNA gene.

The terms "DNA construct" or "synthetic DNA construct" relate to an artificially-designed segment of DNA that can, for example, be used to incorporate genetic material into a target tissue or cell. The terms "vector", "synthetic vector" or "synthetic gene delivery vehicle (GDV)" refer to any means for delivering a nucleic acid, for example a coding nucleic acid together with regulatory elements like promoter sequences and termination signals, into a living cell. Many viral and non-viral vectors are known. Examples are plasmids, viruses, cationic liposomes or polymers etc.

For the term "nucleic acid encoding a transfer RNA" the terms "transfer RNA gene" or "tRNA gene" may also be used synonymously.

The term "5' leader sequence" as used herein refers to a sequence of nucleotides located "upstream" of the 5' end of a tRNA gene, for example approximately 100 nt upstream of the 5' end of the coding sequence for the mature tRNA, comprising a nucleotide sequence functioning as a binding site for the transcription factor TFIIIB. The term "5' leader sequence" refers in particular to the sequence of nucleotides comprising or consisting of positions -100 to -1 5' from the transcription start of a tRNA gene. "Mature tRNA" refers to a fully processed and functional tRNA. The terms "extragenic leader sequence", "5' untranslated region (5' UTR) or "extragenic pol III binding sequence" may also be used here.

The terms "A box" and "B box" refer to intragenic (gene internal) regions, i.e., sequence motifs of a tRNA gene comprising nucleotide sequences to which the transcription factor TFIIIC of the RNA polymerase III binds. The boxes may also be considered as part of a tRNA promoter (so-called type 2 promoter) or promoter elements. The nucleotide sequence of about 10-14 nucleotides in length (Mitra S., Das P., Samadder A., Das S., Betai R., Chakrabarti J., 2015, Eukaryotic tRNAs fingerprint invertebrates vis-à-vis vertebrates, Journal of Biomolecular Structure and Dynamics, doi: 10.1080/07391102.2014.990925) forming the A box are located in the region of the tRNA gene encoding part of the D arm, the nucleotide sequence of about 11 nucleotides in length (Mitra S., Das P., Samadder A., Das S., Betai R., Chakrabarti J., 2015, Eukaryotic tRNAs fingerprint invertebrates vis-à-vis vertebrates, Journal of Biomolecular Structure and Dynamics, doi: 10.1080/07391102.2014.990925) forming the B box are located in the region of the tRNA gene encoding part of the T loop. An 11nt consensus sequence for the B-Box is given by Mitra et al. 2015 (see above) as being RGTTCRANNCY, covering the nucleotides N52-N62 of the mature tRNA. Any numbering in relation to a tRNA gene or part thereof, e.g., the A and B boxes, refers to the nucleotide numbering within the mature tRNA, following the tRNA numbering convention (see below).

The term "encoding" in relation to a nucleotide sequence of a tRNA gene means that the nucleotide sequence is transcribed into a tRNA or part of a tRNA. A direct reference to a tRNA encoded by the DNA construct of the invention, e.g., a reference to a structural part of the mature tRNA, for example, the D arm, anticodon arm, T arm or acceptor stem, is to be understood as referring to the regions of the tRNA gene in the nucleic acid encoding those structural parts, unless clearly stated otherwise or clearly recognizable from the context. A wording according to which an encoded tRNA comprises, for example, a T arm of a specific sequence, the sequence presented as a DNA (with the nucleotides A, C, G, and T), is thus to be understood as referring to the sequence within the tRNA gene coding for the corresponding structure, wherein the corresponding structure appears in the mature tRNA transcribed from the tRNA gene, however, with T replaced by U, and, as the case may be, with modified nucleotides.

The term "sequence motif" or "sequence signature" refers to a specific sequence or consensus sequence having a particular function, e.g., as a binding site for an RNA polymerase.

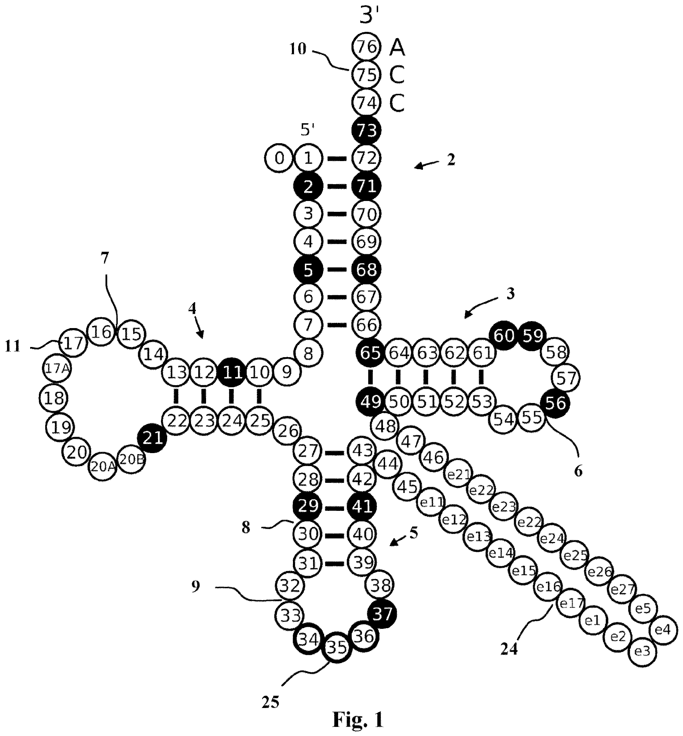

The terms "transfer ribonucleic acid" or "tRNA" refer to RNA molecules with a length of typically 73 to 90 nucleotides, which mediate the translation of a nucleotide sequence in a messenger RNA into the amino acid sequence of a protein. tRNAs are able to covalently bind a specific amino acid at their 3' CCA tail at the end of the acceptor stem, and to base-pair via a usually three-nucleotide anticodon in the anticodon loop of the anticodon arm with a usually three-nucleotide sequence (codon) in the messenger RNA. Some anticodons can pair with more than one codon due to a phenomenon known as wobble base pairing. The secondary "cloverleaf' structure of tRNA comprises the acceptor stem binding the amino acid and three arms ("D arm", "T arm" and "anticodon arm") ending in loops (D loop, T loop (TψC loop), anticodon loop), i.e. sections with unpaired nucleotides. The terms "D stem", "T stem" (or "TψC stem") and "anticodon stem" (also "AC stem") relate to portions of the D arm, T arm and anticodon arm, respectively, with paired nucleotides. Aminoacyl tRNA synthetases charge (aminoacylate) tRNAs with a specific amino acid. Each tRNA contains a distinct anticodon triplet sequence that can base-pair to one or more codons for an amino acid. By convention, the nucleotides of tRNAs are often numbered 1 to 76, starting from the 5'-phosphate terminus, based on a "consensus" tRNA molecule consisting of 76 nucleotides, and regardless of the actual number of nucleotides in the tRNA, which are not always of a length of 76 nt due to variable portions, such as the D loop or the variable loop in the tRNA (see Fig. 1). Following this convention, nucleotide positions 34-36 of naturally occurring tRNA refer to the three nucleotides of the anticodon, and positions 74-76 refer to the terminating CCA tail. Any "supernumerary" nucleotide can be numbered by adding alphabetic characters to the number of the previous nucleotide being part of the consensus tRNA and numbered according to the convention, for example 20a, 20b etc. for the D-arm, or by independently numbering the nucleotides and adding a leading letter, as in case of the variable loop such as e11, e12 etc. (see, for example, Sprinzl M, Horn C, Brown M, Ioudovitch A, Steinberg S. Compilation of tRNA sequences and sequences of tRNA genes. Nucleic Acids Res. 1998;26(1): 148-53). In the following, the tRNA-specific numbering will also be referred to as "tRNA numbering convention" or "transfer RNA numbering convention".

The term "tRNA body" refers to the portion of the tRNA outside the anticodon loop.

The term "intron" relates to a polynucleotide sequence in a nucleic acid that is part of the nucleic acid within a genome and within a first nucleic acid product transcribed from the genomic nucleic acid, but is not contained in the final nucleic acid product. In case of a protein gene, for example, an intron is a non-coding polynucleotide sequence separating coding polynucleotide sequences (exons), which is excised from the pre-mRNA transcribed from the protein gene in a process called "splicing". In case of a transfer RNA, for example, the term intron relates to a polynucleotide sequence that is excised (spliced) from a pre tRNA transcribed from the tRNA gene.

The term "intronic tRNA" relates to a tRNA, the precursor (pre-tRNA) of which contains an intron that is spliced from the pre-tRNA during processing of the pre-tRNA into the final (mature) tRNA. The term "non-intronic tRNA" relates to tRNA being generated from pre-tRNAs that do not contain an intron and do not undergo splicing. The terms "intronic tRNA" or "non-intronic tRNA" encompass the pre-tRNA and the mature tRNA.

The term "tDNA", if used herein, relates to a sequence of DNA encoding a tRNA, in particular a DNA sequence having the sequence of a tRNA wherein the uracil nucleotides (U) of the tRNA are replaced with thymine nucleotides (T).

The terms "engineered transfer ribonucleic acid" or "synthetic tRNA" refer to a tRNA modified by chemical or molecular biological methods or a non-naturally occurring tRNA. The terms "engineered" and "synthetic" are used synonymously here. An example is a tRNA that will be aminoacylated with an amino acid under natural conditions, but has an anticodon pairing to a stop codon instead of the anticodon for the corresponding amino acid.

The term "codon" refers to a sequence of nucleotide triplets, i.e. three DNA or RNA nucleotides, corresponding to a specific amino acid or stop signal during protein synthesis. A list of codons (on mRNA level) and the encoded amino acids are given in the following:

| Amino acid | One Letter Code | Codons |
|---|---|---|
| Ala | A | GCU, GCC, GCA, GCG |
| Arg | R | CGU, CGC, CGA, CGG, AGA, AGG |
| Asn | N | AAU, AAC |
| Asp | D | GAU, GAC |
| Cys | C | UGU, UGC |
| Gln | Q | CAA, CAG |
| Glu | E | GAA, GAG |
| Gly | G | GGU, GGC, GGA, GGG |
| His | H | CAU, CAC |
| Ile | I | AUU, AUC, AUA |
| Leu | L | UUA, UUG, CUU, CUC, CUA, CUG |
| Lys | K | AAA, AAG |
| Met | M | AUG |
| Phe | F | UUU, UUC |
| Pro | P | CCU, CCC, CCA, CCG |
| Ser | S | UCU, UCC, UCA, UCG, AGU, AGC |
| Thr | T | ACU, ACC, ACA, ACG |
| Trp | W | UGG |
| Tyr | Y | UAU, UAC |
| Val | V | GUU, GUC, GUA, GUG |

| | | |
|---|---|---|
| START: AUG STOP: UAA, UGA, UAG, abbreviated "X" | | |

The term "sense codon" as used herein refers to a codon coding for an amino acid. The term "stop codon" or "nonsense codon" refers to a codon, i.e. a nucleotide triplet, of the genetic code not coding for one of the 20 amino acids normally found in proteins and signalling the termination of translation of a messenger RNA.

The term "frameshift mutation" refers to an out-of-frame insertion or deletion (collectively called "indels") of nucleotides with a number not evenly divisible by three. This perturbs the nucleotide sequence decoding which proceeds in steps of three nucleotide bases. The term "-1 frameshift mutation" relates to the deletion of a single nucleotide causing a shift in the reading frame by one nucleotide leading to the first nucleotide of the following codon being read as part of the codon from which the nucleotide has been deleted. Deletion of one nucleotide from the upstream codon along with several triplets (i.e. deletion of 4, 7, 10 etc. nucleotides) is also considered as -1 frameshifting. The term "+1 frameshift mutation" relates to the insertion of a single nucleotide into a triplet, or the deletion of two nucleotides. The result of either event is to shift the reading frame by one nucleotide, such that a nucleotide of an upstream codon is being read as part of a downstream codon. Insertion of one nucleotide along with several triplets (3n+1 nucleotides, n being an integer, i.e. insertion of 4, 7, 10 etc. nucleotides), or deletion of two nucleotides from the upstream codon along with several triplets (i.e. deletion of 5, 8, 11 etc nucleotides) is also considered as +1 frameshifting.

The term "anticodon" refers to a sequence of usually three nucleotides within a tRNA that base-pair (non-covalently bind) to the three bases (nucleotides) of the codon on the mRNA. In a natural tRNA a standard three-nucleotide anticodon is usually represented by the nucleotides at positions 34, 35 and 36. An anticodon may also contain nucleotides with modified bases. The terms "four nucleotide anticodon" or "four base anticodon" relate to an anticodon having four consecutive nucleotides (bases) which pair with four consecutive bases on an mRNA. The terms "quadruplet nucleotide anticodon" or "quadruplet anticodon" may also be used to denote a "four nucleotide anticodon". The terms "five nucleotide anticodon" or "five base anticodon" relate to an anticodon having five consecutive nucleotides (bases) which bind (base-pair) to five consecutive bases on an mRNA. The terms "quintuplet nucleotide anticodon" or "quintuplet anticodon" may also be used to denote a "five nucleotide anticodon".

The term "anticodon arm" refers to a part of the tRNA comprising the anticodon. The anticodon arm is composed of a stem portion ("anticodon stem"), usually consisting of five base pairs (positions 27-31 and 39-43), and a loop portion ("anticodon loop"), i.e. a consecutive sequence of unpaired nucleotides bound to the anticodon stem. The anticodon arm takes the positions 27 to 43, the position numbering following transfer RNA numbering convention.

The term "anticodon loop" refers to the unpaired nucleotides of the anticodon arm containing the anticodon. Naturally occurring tRNAs usually have seven nucleotides in their anticodon loop, three of which pair to the codon in the mRNA.

The term "extended anticodon loop" refers to an anticodon loop with a higher number of nucleotides in the loop than in naturally occurring tRNAs. An extended anticodon loop may, for example, contain more than seven nucleotides, e.g. eight, nine, or ten nucleotides. In particular, the term relates to an anticodon loop comprising an anticodon composed of more than three consecutive nucleotides, e.g. four, five or six nucleotides, being able to base-pair with a corresponding number of consecutive nucleotides in an mRNA.

The term "anticodon stem" refers to the paired nucleotides of the anticodon arm that carry the anticodon loop.

The term "T arm" relates to a portion of a tRNA composed of a stem portion ("T stem") and a loop portion ("T loop") between the acceptor stem and the variable loop. The T arm takes the positions 49 to 65, the position numbering following transfer RNA numbering convention. The T stem is usually composed of five nucleotide pairs (taking positions 49-53 and 61-65).

The term "T stem" (or "TψC" stem) relates to the paired nucleotides of the T arm, which carry the T loop, i.e. the unpaired nucleotides of the T arm.

The term "D arm" relates to the tRNA portion composed of a stem ("D stem"), i.e. the paired nucleotides of the D arm, and a D loop, i.e. the unpaired nucleotides of the D arm, between the acceptor stem and the anticodon arm. The D arm takes the positions 10-25, the position numbering following transfer RNA numbering convention.

The term "variable loop" refers to a tRNA loop located between the anticodon arm and the T arm. The number of nucleotides composing the variable loop may largely vary from tRNA to tRNA. The variable loop can thus be rather short or even missing, or rather large, forming, for example, a helix. The term "variable arm" may synonymously be used for the term "variable loop". Use of the term "variable loop" does not exclude the existence of a "stem" portion within the variable loop, i.e. a stretch of consecutive nucleotides of the variable loop forming base pairs with a complementary stretch of other consecutive nucleotides of the variable loop. The variable loop takes the positions 44 to 48, the position numbering following transfer RNA numbering convention. It should be noted that the variable loop may have a considerable number of additional nucleotides that are separately numbered (see Fig. 1).

The term "acceptor stem" relates to the site of attachment of amino acids to the tRNA. An acceptor stem is often formed by 7 base pairs.

The terms "codon base triplet" or "anticodon base triplet", when used herein, refer to sequences of three consecutive nucleotides which form a codon or anticodon. Synonymously, the terms "three nucleotide codon" (also "three base codon") or "three nucleotide anticodon" (also "three base anticodon"), or abbreviations thereof, e.g. "3nt codon" or "3nt anticodon", may be used.

The term "base pair" refers to a pair of bases, or the formation of such a pair of bases, joined by hydrogen bonds. The term "Watson-Crick base pair" may also be used for such a pair of bases. One of the bases of the base pair is usually a purine, and the other base is usually a pyrimidine. In RNA, for example, the bases adenine and uracil can form a base pair and the bases guanine and cytosine can form a base pair. In DNA thymine usually forms a base pair with adenine instead of uracil. However, the formation of other base pairs ("wobble base pairs") is also possible, e.g. base pairs of guanine-uracil (G-U), hypoxanthine-uracil (I-U), hypoxanthine-adenine (I-A), and hypoxanthine-cytosine (I-C). The term "being able to base-pair" refers to the ability of nucleotides or sequences of nucleotides to form hydrogen-bond-stabilised structures with a corresponding nucleotide or nucleotide sequence. Base pairing can occur intramolecular, e.g., within a single-stranded nucleic acid (e.g., a tRNA), or intermolecular, i.e., between different nucleic acid molecules.

The term "base", as used herein, for example in terms like "the bases A, C, G or U" or "the bases A, C, G or T", encompasses or is synonymously used to the term "nucleotide", unless the context clearly indicates otherwise.

Abbreviations used her for bases or nucleotides are according to the IUPAC nucleotide code and standard ST.26 (version 1.5), as follows:

| IUPAC nucleotide code | Base |
|---|---|
| A | Adenine |
| C | Cytosine |
| G | Guanine |
| T (or U) | Thymine (or Uracil in RNA) |
| R | A or G |
| Y | C or T/U |
| S | G or C |
| W | A or T/U |
| K | G or T/U |
| M | A or C |
| B | C or G or T/U |
| D | A or G or T/U |
| H | A or C or T/U |
| V | A or C or G |
| N | any base |
| . or - | gap |

"PTC" refers to a premature termination codon. This is a stop codon introduced into a coding nucleic acid sequence by a nonsense mutation, i.e. a mutation in which a sense codon coding for one of the twenty proteinogenic amino acids specified by the standard genetic code is changed to a chain-terminating codon. The term thus refers to a premature stop signal in the translation of the genetic code contained in mRNA. The term "premature stop codon" ("PSC") may be used synonymously for a premature termination codon, PTC.

The terms "frameshift suppression" or "frameshift rescue" refer to mechanisms masking the effects of a frameshift mutation and at least partly restoring the wild-type phenotype.

The terms "nonsense suppression", "nonsense mutation suppression", "PTC suppression" or "PTC rescue" refer to mechanisms masking the effects of a nonsense mutation and at least partly restoring mRNA translation and the wild-type phenotype.

The term "tRNA sequestration" relates to the irreversible binding of a tRNA to a tRNA synthetase such that the tRNA binds to the tRNA synthetase, but is not or essentially not released from the tRNA synthetase.

The term "suppressor tRNA" relates to a tRNA altering the reading of a messenger RNA in a given translation system such that, for example, a frameshift or nonsense mutation is "suppressed" and the effects of the frameshift or nonsense mutation are masked and the wild-type phenotype at least partly restored. An example of a suppressor tRNA is a tRNA carrying an amino acid and being able to base-pair to a PTC in an mRNA or, in case of a tRNA with an extended anticodon loop and a four-, five- or six-base anticodon, for example, to a section on an mRNA having two consecutive codons, one or both of which being mutated, or having a first and consecutive second codon, of which one is intact and one has an insertion or deletion. The translation system can thus correct the reading frame in the case of frameshift mutation or read through a PTC in the case of non-sense mutation.

The term "decoding activity" in relation to a tRNA refers to the property or capacity of a transfer RNA to be used by the translation machinery of a cell as an amino acid donor for the production of a protein. A tRNA has a "decoding activity" if, under physiologic conditions, it is charged with a cognate amino acid and the charged tRNA (aminoacyl-tRNA) is subsequently incorporated into a protein. The decoding activity of a first tRNA for a given amino acid can, for example, be compared with the decoding activity of a second tRNA for the same amino acid by comparing the proportion at which the amino acid of the first or second tRNA is incorporated in the protein. The terms "rescue activity", "suppression activity", "suppression efficiency" or "readthrough activity" refers to the decoding activity or efficiency of a suppressor tRNA, e.g. a frameshift suppressor or nonsense suppressor tRNA, in particular a tRNA designed to decode a premature stop codon in an mRNA into an amino acid, such that translation of the mRNA into the corresponding protein is not prematurely interrupted.

A term according to which a tRNA encoded by the DNA construct of the invention "still serves its function in the translational machinery of a living cell" relates to a tRNA transcribed from the tRNA gene having a decoding activity. In case of a suppressor tRNA, the term relates to a tRNA having a rescue activity greater than the spontaneous (stochastic) rescue activity in a given cellular environment.

The term "aminoacylation" relates to the enzymatic reaction in which a tRNA is charged with an amino acid. An aminoacyl tRNA synthetase (aaRS) catalyses the esterification of a specific cognate amino acid or its precursor to a compatible cognate tRNA to form an aminoacyl-tRNA. The term "aminoacyl-tRNA" thus relates to a tRNA with an amino acid attached to it. Each aminoacyl-tRNA synthetase is highly specific for a given amino acid, and, although more than one tRNA may be present for the same amino acid, there is only one aminoacyl-tRNA synthetase for each of the 20 proteinogenic amino acids. The terms "charge" or "load" may also be used synonymously for "aminoacylate".

The term "expression" refers to the conversion of a genetic information into a functional product, for example the formation of a protein or a nucleic acid, e.g. functional RNA (e.g., transfer RNA), on the basis of the genetic information. The term not only encompasses the biosynthesis of a protein, e.g., an enzyme, based on genetic information including previous processes such as transcription or splicing, i.e., the formation of mRNA based on a DNA template, but also the synthesis of a functional RNA molecule, for example a tRNA. In relation to the production of a mature tRNA in a cell from a tRNA gene, in particular in terms of increased or decreased production, the term "transcription" may be used synonymously to the term "expression", thus not only encompassing the synthesis of the first RNA product (i.e., the pre-tRNA) but also the processing of the first RNA product to the final RNA product (i.e., the mature tRNA). The terms "expression strength" or "expression level" relate to the amount of product synthesized from a DNA template. The term "high expression" relates to a high expression level, i.e. an expression level higher than an average expression level observed for a given molecule species, e.g., tRNA. The term "low expression" relates to a low expression level, i.e. an expression level lower than an average expression level observed for a given molecule species, e.g., tRNA.

The tRNA encoded by the nucleic acid, for example a suppressor tRNA, is arranged in the synthetic DNA construct, e.g., a synthetic vector, in a transcribable form, i.e. in a form that it is transcribed under natural conditions once introduced in a living mammalian cell, e.g., human cell, such that the encoded tRNA is produced in the cell, preferably using the natural transcription machinery of the cell. The tRNA may be arranged in a manner that it can be conditionally transcribed, i.e., depending on specific conditions in the cell. The sequence motifs in the 5' leader sequence cause the tRNA arranged downstream from the 5' leader sequence to be more strongly (at a higher level) or more weakly (at a lower level) transcribed than in the absence of these motifs.

The invention provides, in a first embodiment, a DNA construct comprising, functionally linked to the nucleic acid encoding the transfer RNA, three sequence motifs, designated I_{H} (TGACCTAAGTGTAAAGT, SEQ ID NO: 1), II_{H} (TGAGATTTCCTTCAGGTT, SEQ ID NO: 2) and III_{H} (TATATAGTTCTGTATGAGACCACTCTTTCCC, SEQ ID NO: 3), promoting high expression of the tRNA, and three sequence motifs, I_{L} (ACCATAAACGTGAAATG, SEQ ID NO: 4), II_{L} (TCTTTGGATTTGGGAATC, SEQ ID NO: 5) and III_{L} (TTATAAGTTCTGTATGAGACCACTCTTTCCC, SEQ ID NO: 6), promoting low expression of the tRNA, when present in the 5' leader sequence of the tRNA encoded in the DNA construct. The sequence motifs can be included in a 5' leader sequence of an associated tRNA gene in order to control the level of expression of the tRNA.

In preferred embodiments of this first embodiment of a DNA construct of the invention, the sequence motifs I_{H} and I_{L} are not both present in the same 5' leader sequence. The same applies to the sequence motifs II_{H} and II_{L} and to the sequence motifs III_{H} and III_{L}. Therefore, in a preferred embodiment of the synthetic DNA construct of the invention,
the 5' leader sequence does not comprise the sequence motif I_{H} if it comprises the sequence motif I_{L}, and vice versa,
ii. the 5' leader sequence does not comprise the sequence motif II_{H} if it comprises the sequence motif II_{L}, and vice versa, and
iii. the 5' leader sequence does not comprise the sequence motif III_{H} if it comprises the sequence motif III_{L}, and vice versa.

In a preferred embodiment of the first embodiment of the synthetic DNA construct of the invention, the sequence motifs I_{L}, II_{L}, III_{L}, I_{H}, II_{H} or III_{H}, if present, are arranged upstream of the tRNA encoding nucleic acid at specific positions. In particular, the sequence motifs I_{L} and I_{H} are, if present, arranged in such a manner that they occupy positions -66 to -50, the positions relating to the region upstream of the nucleic acid encoding the tRNA . The sequence motifs II_{L} and II_{H} are, if present, arranged in such a manner that they occupy positions -49 to -32, and the sequence motifs III_{L} and III_{H} are, if present, arranged in such a manner that they occupy positions -31 to -1. In a preferred embodiment of the synthetic DNA construct according to the invention, the sequence motifs are thus preferably arranged such that
i. if the 5' leader sequence comprises the sequence motif I_{H} or I_{L}, the sequence motif I_{H} or I_{L} has, in 5'-3' direction, the positions -66 to -50,
ii. if the 5' leader sequence comprises the sequence motif II_{H} or II_{L}, the sequence motif II_{H} or II_{L} has, in 5'-3' direction, the positions -49 to -32, and
iii. if the 5' leader sequence comprises the sequence motif III_{H} or III_{L}, the sequence motif III_{H} or III_{L} has, in 5'-3' direction, the positions -31 to -1.

In a preferred embodiment of the first embodiment of the synthetic DNA construct of the invention, the 5' leader sequence comprises at least two sequence motifs selected from the sequence motifs I_{L}, II_{L}, III_{L}, I_{H}, II_{H} or III_{H}, wherein the at least two sequence motifs are arranged, in 5'-3' direction, in the order I_{H} - II_{H}, II_{H} - III_{H}, I_{H} - III_{H}, I_{L} - II_{L}, II_{L} - III_{L}, I_{L} - III_{L}, I_{H} - II_{L}, I_{H} - III_{L}, II_{H} - III_{L}, I_{L} - II_{H}, II_{L} - III_{H}, or I_{L} - III_{H}.

As mentioned above, the sequence motifs are preferably arranged such that they take the positions mentioned, i.e., positions -66 to -50 for sequence motifs I_{L} and I_{H}, positions -49 to -32 for sequence motifs II_{L} and II_{H}, and positions -31 to -1 for sequence motifs III_{L} and III_{H}.

In a further preferred embodiment of the synthetic DNA construct according to the invention, the 5' leader sequence comprises three sequence motifs selected from the sequence motifs I_{L}, II_{L}, III_{L}, I_{H}, II_{H} or III_{H}. Preferably, the sequence motifs are arranged in the following order, in 5'-3' direction: I_{L} - II_{L} - III_{L}, I_{H} - II_{H} - III_{H}, I_{H} - II_{L} - III_{L}, I_{L} - II_{H} - III_{L}, I_{L} - II_{L} - III_{H}, I_{H} - II_{H} - III_{L}, I_{H} - II_{L} - III_{H}, or I_{L} - II_{H} - III_{H}. Preferably, the sequence motifs are arranged directly one after the other, i.e., without nucleotides or linkers in between. Again, the sequence motifs preferably have the positions mentioned above.

In preferred embodiments of the first embodiment of the synthetic DNA construct of the invention, the 5' leader sequence may have one of the sequences according to SEQ ID NOs: 12-37, according to the following list:
P1 (I_{H}-II_{H}-III_{H}, SEQ ID NO: 12): 
TGACCTAAGTGTAAAGTTGAGATTTCCTTCAGGTTTATATAGTTCTGTATGAGACCA
CTCTTTCCC
P2 (I_{L}-II_{L}-III_{L}, SEQ ID NO: 13): 
ACCATAAACGTGAAATGTCTTTGGATTTGGGAATCTTATAAGTTCTGTATGAGACC
ACTCTTTCCC
P3 (I_{H}, SEQ ID NO: 14): 
TGACCTAAGTGTAAAGTNNNNNNNNNNNNNNNNNNNNNNNNNNNNNNNNNNNNNNNNNNNNNNNN
NNNNNNNNNNNNNN
P4 (II_{H}, SEQ ID NO: 15): 
NNNNNNNNNNNNNNNNNNTGAGATTTCCTTCAGGTTNNNNNNNNNNNNNNNNNNNNN

NNNNNNNNNNNNN
P5 (III_{H}, SEQ ID NO: 16): 
NNNNNNNNNNNNNNNNNNNNNNNNNNNNNNNNNNNTATATAGTTCTGTATGAGA
CCACTCTTTCCC
P6 (I_{H}-II_{H}, SEQ ID NO: 17): 
TGACCTAAGTGTAAAGTTGAGATTTCCTTCAGGTTNNNNNNNNNNNNNNNNNNNNN
NNNNNNNNNNN
P7 (II_{H}-III_{H}, SEQ ID NO: 18): 
NNNNNNNNNNNNNNNNNNNNTGAGATTTCCTTCAGGTTTATATAGTTCTGTATGAGACC
ACTCTTTCCC
P8 (I_{H}-III_{H}, SEQ ID NO: 19): 
TGACCTAAGTGTAAAGTNNNNNNNNNNNNNNNNNNTATATAGTTCTGTATGAGAC

CACTCTTTCCC
P9 (I_{L}, SEQ ID NO: 20): 
ACCATAAACGTGAAATGNNNNNNNNNNNNNNNNNNNNNNNNNNNNNNNNNNNNNNN

NNNNNNNNNNNNN
P10 (II_{L}, SEQ ID NO: 21): 
NNNNNNNNNNNNNNNNNTCTTTGGATTTGGAATCNNNNNNNNNNNNNNNNNNNNNN

NNNNNNNNNNNNN
P11 (III_{L}, SEQ ID NO: 22): 
NNNNNNNNNNNNNNNNNNNNNNNNNNNNNNNNNNNNNNNNNTTATAAGTTCTGTATGAGA
CCACTCTTTCCC
P12 (I_{L}-II_{L}, SEQ ID NO: 23): 
ACCATAAACGTGAAATGTCTTTGGATTTGGGAATCNNNNNNNNNNNNNNNNNNNNNNNNNNNN

NNNNNNNNNNNN
P13 (II_{L}-III_{L}, SEQ ID NO: 24): 
NNNNNNNNNNNNNNNNNNNNTCTTTGGATTTGGAATCTTATAAGTTCTGTATGAGACC
ACTCTTTCCC
P14 (SEQ ID NO: 25): 
ACCATAAACGTGAAATGNNNNNNNNNNNNNNNNNNTTATAAGTTCTGTATGAGAC
CACTCTTTCCC
P15 (SEQ ID NO: 26): 
TGACCTAAGTGTAAAGTTCTTTGGATTTGGGAATCNNNNNNNNNNNNNNNNNNNNNNNN

NNNNNNNNNNN
P16 (SEQ ID NO: 27): 
TGACCTAAGTGTAAAGTNNNNNNNNNNNNNNNNNNNNNNNNNNNNNNNNNNNNNNNNTTATAAGTTCTGTATGAGAC
CACTCTTTCCC
P17 (SEQ ID NO: 28): 
NNNNNNNNNNNNNNNNNNNNTGAGATTTCCTTCAGGTTTTATAAGTTCTGTATGAGACC
ACTCTTTCCC
P18 (SEQ ID NO: 29): 
ACCATAAACGTGAAATGTGAGATTTCCTTCAGGTTNNNNNNNNNNNNNNNNNNNNNNN

NNNNNNNNNNN
P19 (SEQ ID NO: 30): 
NNNNNNNNNNNNNNNNNNNNTCTTTGGATTTGGGAATCTATATAGTTCTGTATGAGACC
ACTCTTTCCC
P20 (SEQ ID NO: 31): 
ACCATAAACGTGAAATGNNNNNNNNNNNNNNNNNNTATATAGTTCTGTATGAGAC

CACTCTTTCCC
P21 (SEQ ID NO: 32): 
TGACCTAAGTGTAAAGTTCTTTGGATTTGGGAATCTTATAAGTTCTGTATGAGACCA
CTCTTTCCC
P22 (SEQ ID NO: 33): 
ACCATAAACGTGAAATGTGAGATTTCCTTCAGGTTTTATAAGTTCTGTATGAGACC
ACTCTTTCCC
P23 (SEQ ID NO: 34): 
ACCATAAACGTGAAATGTCTTTGGATTTGGGAATCTATATAGTTCTGTATGAGACC
ACTCTTTCCC
P24 (SEQ ID NO: 35): 
TGACCTAAGTGTAAAGTTGAGATTTCCTTCAGGTTTTATAAGTTCTGTATGAGACCA
CTCTTTCCC
P25 (SEQ ID NO: 36): 
TGACCTAAGTGTAAAGTTCTTTGGATTTGGGAATCTATATAGTTCTGTATGAGACCA
CTCTTTCCC
P26 (SEQ ID NO: 37):

ACCATAAACGTGAAATGTGAGATTTCCTTCAGGTTTATATAGTTCTGTATGAGACC
ACTCTTTCCC

N stands for any nucleotide (A, G, C or T).

In a second embodiment of the synthetic DNA construct of the invention, the 5' leader sequence comprises one sequence motif selected from the group consisting of the sequence motifs VNANANTVHANANNTTNNNATRANATTCNGDGVGNAANATABTNCTVGND, designated 50nt_{H}, (SEQ ID NO: 7) and
GCNGGDGGCGNGTTCBBCTGTNVNAGCNTNCGDNGNCGTTTNNCNTCGGT, designated 50nt_{L}, (SEQ ID NO: 8).

The abbreviations have the standard meaning as indicated above (see, e.g., ST.26 ver. 1.5).

The sequence motif 50nt_{H} promotes a higher expression of the downstream tRNA gene, and 50nt_{L} causes a lower expression of the downstream tRNA gene.

In a preferred embodiment of this second embodiment of the synthetic DNA construct of the invention the 5' leader sequence does not comprise the sequence motif 50nt_{H} if it comprises the sequence motif 50nt_{L}, and vice versa.

Further preferred, in this second embodiment, the sequence motifs 50nt_{H} or 50nt_{L} have the positions -50 to -1, i.e., if the sequence of 50nt_{H} is present, it preferably takes, in 5'-3' direction, the positions -50 to -1, and if the sequence of 50nt_{L} is present, it takes, in 5'-3' direction, positions -50 to -1.

The sequence motif 50nt_{H} may have one of the following sequences:
P27 (50nt_{H1}, SEQ ID NO: 38)
   CCATGATCCCCCACTATTAAGGATATCCGGAGAGGATGCTACCTATCAGG
P28 (50nt_{H2}, SEQ ID NO: 39)
   AGACCAGCTGTATAGCCTCAGAATGATCCGTCCGGAAACCCTTACTAGGT
P29 (50nt_{H3}, SEQ ID NO: 40)
   GCATATTGCAGAACTTGTGAAGAGGCTTTATGCGCCACACACTGCAAGCA
P30 (50nt_{H4}, SEQ ID NO: 41)
   GCAAAAGCAAAAGCATTAAGTAGCTTTCTGGCATAAACATATTGCAGCTG
P31 (50nt_{H5}, SEQ ID NO: 42)
   GGTTGCGGTAAGCATTAGAGGGCTATCAGCAGCATCTTATCGCAGCGGAG
P32 50nt_{H6}, (SEQ ID NO: 43)
   CTGCAGTATAACCTTGAAGTACCATCACGGAGAGAAACAGTGCCATCTCA
P33 (50nt_{H7}, SEQ ID NO: 44)
   AACGACTACGTAGTGTTCTATAACAATCATGAGAAATTTTAGTTCTAGAT

The sequence motif 50nt_{L} may have one of the following sequences:
P34 (50nt_{L1}, SEQ ID NO: 45)
   GCTGGTGGCGAGTTCCGCTGTGCCAGCTTCCGTTGGCGTTTGCCATCGGT
P35 (50nt_{L2}, SEQ ID NO: 46)
   GCTGGTGGCGAGTTCCGCTGTGCCAGCTTCCGTTGGCGTTTGCCATCGGT
P36 (50nt_{L3}, SEQ ID NO: 47)
   GCTGGTGGCGAGTTCCGCTGTGCCAGCTTCCGTTGGCGTTTGCCATCGGT
P37 (50nt_{L4}, SEQ ID NO: 48)
   ATGCGGCCTTGTGTGGTGGCTCATTGTGGTGGGCCAGAAAACGCGTGCAA
P38 (50nt_{L5}, SEQ ID NO: 49)
   GCCAGAGGCGCTGGGGCCAGGAGGCGCAAGCCGGGCCCTGAAGCCGCCTC
P39 (50nt_{L6}, SEQ ID NO: 50)
   AAAGAAATTAGGAAATTCCTGTGGAAGCTGGCGCGTTGATGCACTTCGTC
P40 (50nt_{L7}, SEQ ID NO: 51)
   CAACAAACATTTTGCTTTTTTAAAATTGAAAGAACAACTGTTTTCCGGGT

In a third embodiment of the synthetic DNA construct of the invention, the 5' leader sequence of the nucleic acid encoding a transfer RNA comprises at least one sequence motif selected from the sequence motifs GAAATGCCTT, designated 10nt_{H1}, (SEQ ID NO: 9), GTGGGAACTA, designated 10nt_{H2}, (SEQ ID NO: 10), and GTGTTGCTTG, designated 10nt_{H3}, (SEQ ID NO: 11). The sequence motifs, designated 10nt_{H1}, 10nt_{H2} and 10nt_{H3}, can advantageously be used to promote higher expression of the tRNA gene arranged downstream of the 5' leader sequence within the synthetic DNA construct. The motifs are preferably arranged within a region from -100 to -1 in relation to the tRNA gene.

Preferably, the 5' leader sequence, in this third embodiment of a synthetic DNA construct of the invention, comprises at least two sequence motifs selected from the sequence motifs 10nt_{H1}, 10nt_{H2} and 10nt_{H3}. Preferably, the at least two sequence motifs are:
i. GAAATGCCTT, 10nt_{H1}, (SEQ ID NO: 9) and GTGTTGCTTG, 10nt_{H3}, (SEQ ID NO: 11), wherein the sequence motifs are arranged, in 5'-3' direction, in the following order: GTGTTGCTTG (SEQ ID NO: 11) - GAAATGCCTT (SEQ ID NO: 9), i.e., 10nt_{H3} - 10nt_{H1}, or
ii. GAAATGCCTT, 10nt_{H1}, (SEQ ID NO: 9) and GTGGGAACTA, 10nt_{H2}, (SEQ ID NO: 10), wherein the sequence motifs are arranged, in 5'-3' direction, in the following order: GAAATGCCTT (SEQ ID NO: 9) - GTGGGAACTA (SEQ ID NO: 10), i.e., 10nt_{H1} - 10nt_{H2}, or
iii. GTGGGAACTA, 10nt_{H2}, (SEQ ID NO: 10), and GTGTTGCTTG, 10nt_{H3}, (SEQ ID NO: 11), wherein the sequence motifs are arranged, in 5'-3' direction, in the following order: GTGTTGCTTG (SEQ ID NO: 11) - GTGGGAACTA (SEQ ID NO: 10), i.e., 10nt_{H3} - 10nt_{H2}.

Further preferred, in this third embodiment of a synthetic DNA construct of the invention, the 5' leader sequence comprises all three sequence motifs GAAATGCCTT (SEQ ID NO: 9), 10nt_{H1}, GTGGGAACTA (SEQ ID NO: 10), 10nt_{H2}, and GTGTTGCTTG (SEQ ID NO: 11), 10nt_{H3}, preferably in the following order, in 5'-3' direction: GTGTTGCTTG (SEQ ID NO: 11) - GAAATGCCTT (SEQ ID NO: 9) - GTGGGAACTA (SEQ ID NO: 10), i.e., 10nt_{H3} - 10nt_{H1} - 10nt_{H2}.

The tRNA encoded by the nucleic acid may be a naturally occurring tRNA, or an engineered, for example microbiologically engineered, tRNA. Preferably the tRNA is engineered, for example, in that the tRNA has an altered anticodon, e.g., an anticodon base-pairing to a stop codon in an mRNA, or has an extended anticodon loop comprising, e.g., a four-, five-, or six-base anticodon, or has a modified D arm, Anticodon arm, T arm or acceptor stem.

In a preferred embodiment of the synthetic DNA construct according to the invention, the nucleic acid encoding the tRNA comprises a B box comprising the sequence H₅₄T₅₅C₅₆G₅₇A₅₈N₅₉T₆₀, preferably, T₅₄T₅₅C₅₆G₅₇A₅₈N₅₉T₆₀, the index representing the positions of the nucleotides in the transfer RNA, the position numbering following transfer RNA numbering convention. N stands for any nucleotide (A, C, G or T), H stands for A, C, or T. Nucleotides with number 54-60 are, in the mature tRNA, arranged within the T arm of the mature tRNA, and form the T loop. A tRNA comprising a B box having the above sequence is transcribed more strongly than a tRNA lacking such a B box. The encoded tRNA may be a natural or synthetic (engineered) transfer RNA.

The transfer RNA encoded by the nucleic acid may have a "normal" anticodon arm, i.e. an anticodon arm including 10 nucleotides forming the anticodon stem and 7 nucleotides forming the anticodon loop containing a three-base anticodon base-pairing with a respective codon on an mRNA. The anticodon can, for example, be designed to base-pair to a premature stop codon on an mRNA, and function as a suppressor tRNA. Alternatively, the encoded transfer RNA can, however, also have an extended anticodon loop having a four-base, five-base or six-base anticodon (see WO 2019/175316 A1, WO 2020/208169 A1, the disclosures of which are incorporated by reference herein in their entirety).

In an embodiment, the nucleic acid encoding the transfer RNA may also contain one or more introns. These intron(s) may be natural occurring introns. In preferred embodiments the intron(s) is (are) inserted into a nucleic acid encoding a tRNA that does not have an intron naturally. However, the tRNA, e.g., suppressor tRNA, encoded by the nucleic acid can also be a tRNA comprising a tRNA body of a tRNA the gene or pre-tRNA of which naturally contains one or more introns, which are, however, preferably different from the introns inserted in the nucleic acid according to the invention. Inclusion of one or more introns allows, at least in some applications, for close to natural tRNA transcription with factors (enzymes) that co-transcriptionally modify tRNAs which in turn leads to more stable output as functional tRNA.

In preferred embodiments, the encoded tRNA may be structurally modified in that the tRNA has a structurally modified D arm, anticodon arm, variable loop and/or T arm. "Structurally modified" means that the mature tRNA contains an anticodon arm, and/or a variable loop and/or a T-arm and/or a D arm that has been modified, i.e. has a nucleotide sequence, for example of the stem portion or the loop portion, that differs from the D arm and/or the anticodon arm and/or the variable loop and/or the T arm of a comparable naturally occurring tRNA. "Comparable" tRNAs are tRNA being aminoacylated by the same aminoacyl-tRNA synthetases. Particular preferred, the encoded tRNA contains, in its mature form, a D arm, an anticodon arm and/or a variable loop and/or a T arm that does not occur in natural tRNAs. It is to be noted here that the invention also relates to the tRNAs, as described below.

The anticodon arm of the mature tRNA encoded by the nucleic acid may, for example, have one of the following general structures with a modified stem portion:
5'-GCAGG-AC-Loop-CCTGT-3'
5'-TTGGG-AC-Loop-CTCAA-3'
5'-TTGGA-AC-Loop-TTCAA-3'
5'-ATGGT-AC-Loop-ACCAT-3'
5'-GCGGA-AC-Loop-TCCGC-3'
5 '-GCGGT-AC-Loop-ACCGC-3'
5 '-GGCGG-AC-Loop-CCGCC-3'
5 '-GGCGC-AC-Loop-GCGCC-3'
5'-TTGGG-AC-loop-CCCAA-3'
5'-CTGGA-AC-loop-TCCAG-3'
5'-CCGGA-AC-loop-TCCGG-3'
5'-GCTGC-AC-loop-GCAGT-3'

Preferred anticodon (AC) arms of the encoded tRNA may have the following general sequences:

| | |
|---|---|
| GCAGGNNNNNNNCCTGT | (SEQ ID NO: 52) |
| GCAGGNNNNNNNNCCTGT | (SEQ ID NO: 53) |
| GCAGGNNNNNNNNNNCCTGT | (SEQ ID NO: 54) |
| GCAGGNNNNNNNNNNCCTGT | (SEQ ID NO: 55) |
| TTGGGNNNNNNNCTCAA | (SEQ ID NO: 56) |
| TTGGGNNNNNNNNCTCAA | (SEQ ID NO: 57) |
| TTGGGNNNNNNNNNCTCAA | (SEQ ID NO: 58) |
| TTGGGNNNNNNNNNNCTCAA | (SEQ ID NO: 59) |
| TTGGANNNNNNNTTCAA | (SEQ ID NO: 60) |
| TTGGANNNNNNNNTTCAA | (SEQ ID NO: 61) |
| TTGGANNNNNNNNNTTCAA | (SEQ ID NO: 62) |
| TTGGANNNNNNNNNNTTCAA | (SEQ ID NO: 63) |
| ATGGTNNNNNNNACCAT | (SEQ ID NO: 64) |
| ATGGTNNNNNNNNACCAT | (SEQ ID NO: 65) |
| ATGGTNNNNNNNNNACCAT | (SEQ ID NO: 66) |
| ATGGTNNNNNNNNNNACCAT | (SEQ ID NO: 67) |
| GCGGANNNNNNNTCCGC | (SEQ ID NO: 68) |
| GCGGANNNNNNNNTCCGC | (SEQ ID NO: 69) |
| GCGGANNNNNNNNNTCCGC | (SEQ ID NO: 70) |
| GCGGANNNNNNNNNNTCCGC | (SEQ ID NO: 71) |
| GCGGTNNNNNNNACCGC | (SEQ ID NO: 72) |
| GCGGTNNNNNNNNACCGC | (SEQ ID NO: 73) |
| GCGGTNNNNNNNNNACCGC | (SEQ ID NO: 74) |
| GCGGTNNNNNNNNNNACCGC | (SEQ ID NO: 75) |
| GGCGGNNNNNNNCCGCC | (SEQ ID NO: 76) |
| GGCGGNNNNNNNNCCGCC | (SEQ ID NO: 77) |
| GGCGGNNNNNNNNNCCGCC | (SEQ ID NO: 78) |
| GGCGGNNNNNNNNNNCCGCC | (SEQ ID NO: 79) |
| GGCGCNNNNNNNGCGCC | (SEQ ID NO: 80) |
| GGCGCNNNNNNNNGCGCC | (SEQ ID NO: 81) |
| GGCGCNNNNNNNNNGCGCC | (SEQ ID NO: 82) |
| GGCGCNNNNNNNNNNGCGCC | (SEQ ID NO: 83) |

Underlined N's represent a 3nt, 4nt, 5nt or 6nt anticodon. N = A, C, G or T, or any modified base (in the final tRNA).

In preferred embodiments, the encoded tRNA comprises, for example, an anticodon (AC) arm of one of the following sequences:

| | |
|---|---|
| GCAGGCTNNNAACCTGT | (SEQ ID NO: 84) |
| GCAGGCTNNNNAACCTGT | (SEQ ID NO: 85) |
| GCAGGCTNNNNNAACCTGT | (SEQ ID NO: 86) |
| GCAGGCTNNNNNNAACCTGT | (SEQ ID NO: 87) |
| TTGGGCTNNNAACTCAA | (SEQ ID NO: 88) |
| TTGGGCTNNNNAACTCAA | (SEQ ID NO: 89) |
| TTGGGCTNNNNNAACTCAA | (SEQ ID NO: 90) |
| TTGGGCTNNNNNNA ACTCAA | (SEQ ID NO: 91) |
| TTGGACTNNNAATTCAA | (SEQ ID NO: 92) |
| TTGGACTNNNNAATTCAA | (SEQ ID NO: 93) |
| TTGGACTNNNNNAATTCAA | (SEQ ID NO: 94) |
| TTGGACTNNNNNNAATTCAA | (SEQ ID NO: 95) |
| ATGGTCTNNNAAACCAT | (SEQ ID NO: 96) |
| ATGGTCTNNNNAAACCAT | (SEQ ID NO: 97) |
| ATGGTCTNNNNNAAACCAT | (SEQ ID NO: 98) |
| ATGGTCTNNNNNNAAACCAT | (SEQ ID NO: 99) |
| GCGGACTNNNAATCCGC | (SEQ ID NO: 100) |
| GCGGACTNNNNAATCCGC | (SEQ ID NO: 101) |
| GCGGACTNNNNNAATCCGC | (SEQ ID NO: 102) |
| GCGGACTNNNNNNAATCCGC | (SEQ ID NO: 103) |
| GCGGTCTNNNAAACCGC | (SEQ ID NO: 104) |
| GCGGTCTNNNNAAACCGC | (SEQ ID NO: 105) |
| GCGGTCTNNNNNAAACCGC | (SEQ ID NO: 106) |
| GCGGTCTNNNNNNAAACCGC | (SEQ ID NO: 107) |
| GGCGGCTNNNAACCGCC | (SEQ ID NO: 108) |
| GGCGGCTNNNNAACCGCC | (SEQ ID NO: 109) |
| GGCGGCTNNNNNAACCGCC | (SEQ ID NO: 110) |
| GGCGGCTNNNNNNAACCGCC | (SEQ ID NO: 111) |
| GGCGCCTNNNAAGCGCC | (SEQ ID NO: 112) |
| GGCGCCTNNNNAAGCGCC | (SEQ ID NO: 113) |
| GGCGCCTNNNNNAAGCGCC | (SEQ ID NO: 114) |
| GGCGCCTNNNNNNAAGCGCC | (SEQ ID NO: 115) |

The underlined N's represent a 3nt, 4nt, 5nt or 6nt anticodon. N = A, C, G or T, or any modified base. A preferred 3nt anticodon is, for example, TCA for a tRNA acting as a PTC suppressor tRNA.

In a further preferred embodiment of the invention, the encoded tRNA comprises, in combination with one of the AC arms mentioned above, or alone, i.e. not in combination with one of the AC arms mentioned above, a variable loop (V loop) having one of the following sequences:
TGGGGTTTCCCC (SEQ ID NO: 116)
AGGGGAAACCCC (SEQ ID NO: 117)

In a further preferred embodiment of the invention, the encoded tRNA of the invention comprises, in combination with one of the AC arms described above and/or in combination with one of the V loops described above, or alone, i.e. not in combination with one of the AC arms mentioned above and/or not in combination with one of the V loops mentioned above, a T arm having the following general sequence:
GCGGG-T-Loop-CCCGT
ACGGG-T-Loop-CCCGT
GTAGG-T-Loop-CCCAT
GTCGG-T-Loop-CCCGT
GGCGG-T-Loop-CCGGT
GCAGG-T-Loop-CCCGT
GCCGG-T-Loop-CCGGT

Preferably, the T-Loop (positions 54-60 according to tRNA numbering convention) has the sequence HTCGANT, H being A, C, or T, i.e., CTCGANT, ATCGANT or TTCGANT, further preferred TTCGANT, for example TTCGAAT or TTCGAGT, preferably TTCGAGT.

In preferred embodiments, the encoded tRNA of the invention comprises, for example, a T arm of one of the following sequences

| | |
|---|---|
| GCGGG*TTCGAAT*CCCGT | (SEQ ID NO: 118) |
| ACGGG*TTCGAAT*CCCGT | (SEQ ID NO: 119) |
| GCGGG*TTCGAGT*CCCGT | (SEQ ID NO: 120) |
| ACGGG*TTCGAGT*CCCGT | (SEQ ID NO: 121) |
| GTAGG*TTCGAGT*CCCAT | (SEQ ID NO: 122) |
| GTCGG*TTCGAGT*CCGAT | (SEQ ID NO: 123) |
| GCCGG*TTCGAGT*CCGGT | (SEQ ID NO: 124) |
| GCAGG*TTCGAGT*CCCGT | (SEQ ID NO: 125) |
| GCCGG*TTCGAGT*CCGGT | (SEQ ID NO: 126) |

The loop part is in italics. The T arm has a stem of five base pairs.

In a further preferred embodiment of the invention, the encoded tRNA of the invention comprises, in combination with one of the AC arms described above and/or in combination with one of the V loops described above and/or in combination with one of the T arms described above, or alone, i.e. not in combination with one of the AC arms mentioned above and/or not in combination with one of the V loops mentioned above and/or not in combination with one of the T arms described above, an acceptor stem having the following general sequence:
5'-GGCTCTG-rest tRNA-CAGAGTC-3'
5'-GGCGCTG-rest tRNA-CAGCGTC-3'
5'-GGCGCGG-rest tRNA-CGGCGTC-3'

The term "rest tRNA" refers to the part of the tRNA between the 5' and 3' portions of the acceptor stem, and includes the D arm, V loops and T arm.

As mentioned above, for a given tRNA encoded by the synthetic DNA construct of the invention, the anticodon arm or at least the anticodon stem, the V loop, the T arm, or at least the T stem, and the acceptor stem can be independently selected from the anticodon arms/stems, V loops, T arms/stems and acceptor stems mentioned above. The encoded tRNA may thus only include one of the anticodon arms/stems, V loops, T arms/stems or acceptor stems mentioned above, for example only an anticodon arm with the sequence of SEQ ID NO: 88, or any combination thereof, for example an anticodon arm with the sequence of SEQ ID NO: 112 and a T arm having the sequence of SEQ ID NO: 120, or an anticodon arm with the sequence of SEQ ID NO: 88 and an acceptor stem as described above.

The skilled person is aware of the fact that a tRNA is aminoacylated with a specific amino acid by a specific aminoacyl tRNA synthetase (aaRS), and that the aaRS is able to recognize its cognate tRNA through unique identity elements at the acceptor stem and/or anticodon loop of the tRNA. In order to include in the DNA construct of the invention a tRNA gene of a tRNA which is, in its mature form, loaded with its cognate amino acid in vivo, the skilled person will design the encoded tRNA with suitable unique identity elements.

In the transfer RNA encoded in the synthetic DNA construct of the invention the anticodon loop may be extended by a large enough number of nucleotides to accommodate a four-, five- or six-base anticodon. The anticodon loop of a mature transfer RNA encoded by the synthetic vector of the invention may, for example, consist of 7-12, preferably 7 to 10 or 8-10, further especially preferred 8 or 9 nucleotides.

The synthetic DNA construct can, for example, be a synthetic vector, and thus be, for example, any suitable biological nucleic acid delivery vehicle, in particular a delivery vehicle suitable for delivery of a nucleic acid into a living mammalian cell, e.g., a human cell. Suitable vehicles include, for example, viral vectors such as adeno-associated virus (AAV)-based viral vectors, encapsulation in or coupling to nanoparticles, e.g., lipid nanoparticles, and others. Preferably, the vector is a viral vector. Viral vectors include, for example, adeno-associated (AAV) viruses, adenoviruses (e.g., AAV3, AAV8, or AAV9), retroviruses (see, for example, Bulcha, J.T., Wang, Y., Ma, H. et al. Viral vector platforms within the gene therapy landscape. Sig Transduct Target Ther 6, 53, 2021, doi: 10.1038/s41392-021-00487-6; Kotterman, M.A., Chalberg, T.W., Schaffer, D.V., 2015, Viral Vectors for Gene Therapy: Translational and Clinical Outlook, Annu. Rev. Biomed. Eng. 2015. 17:63-89, 10.1146/annurev-bioeng-071813-104938).

In a second aspect the invention relates to a pharmaceutical composition comprising the synthetic DNA construct according to the invention, and a pharmaceutically acceptable carrier. An example of a simple carrier is a buffer solution or a physiologic salt solution.

In a further aspect the invention relates to the synthetic DNA construct according to the first aspect of the invention, or the pharmaceutical composition according to the second aspect of the invention for use as a medicament. The synthetic DNA construct or pharmaceutical composition of the invention is especially useful for treating patients with a disease associated with a nonsense mutation, i.e. a premature termination codon (PTC), or a frameshifting causing the absence of a functional protein or the dysfunction of a protein. Examples for diseases, in which the tRNA of the invention may advantageously be employed are Hurler syndrome (MPS I, ICD code E76.0, beta-thalassemia (ICD-10 code D56.1), neurofibromatosis type 1 (NF1, ICD-10 code Q85.0), Duchenne muscular dystrophy (DMD, ICD-10 code G71.0, Crohn's disease (CD, ICD-10 code K50), cystic fibrosis (CF, ICD-10 code E84), neuronal ceroid lipofuscinosis (NCL, ICD-10 code E75.4) and Tay-Sachs disease (TSD, ICD-10 code E75.0). The DNA construct, e.g., vector, of the invention is also useful for treating patients with a disease which is at least partly caused by the sequestration of a tRNA leading to depletion of the cellular pool of the tRNA, e.g. Charcot-Marie-Tooth disease (CMT disease, ICD-10 code DG600).

The DNA construct or pharmaceutical composition of the invention may, for example, also advantageously be used for treating a disease, e.g. hereditary motor and sensory neuropathy (Charcot-Marie-Tooth (CMT) disease), in which the natural tRNA is sequestered due to, for example, mutations in aminoacyl-tRNA-synthetase, and thus not or not sufficiently available for the translation machinery of the cell..

In a further aspect, the invention relates to a method of treating a person having a disease associated with a nonsense (PTC) or frameshift mutation, comprising administering an effective amount of the synthetic DNA construct of the invention or of a pharmaceutical composition comprising the synthetic DNA construct of the invention to the person. In a preferred embodiment the method is for treating Hurler syndrome (MPS I, ICD code E76.0), beta-thalassemia (ICD-10 code D56.1), neurofibromatosis type 1 (NF1, ICD-10 code Q85.0), Duchenne muscular dystrophy (DMD, ICD-10 code G71.0), Crohn's disease (CD, ICD-10 code K50), cystic fibrosis (CF, ICD-10 code E84), neuronal ceroid lipofuscinosis (NCL, ICD-10 code E75.4) or Tay-Sachs disease (TSD, ICD-10 code E75.0).

In a further aspect, the invention also relates to any of the structurally modified tRNAs, as described above, in the form of mature tRNA. In the tRNAs, the nucleotide thymine has to be replaced with the nucleotide Uracil.

In a still further aspect, the invention also relates to a synthetic DNA construct, consisting of or comprising one of the encoded modified tRNAs described above. Since the tRNAs are encoded in a DNA, the encoded tRNA may also be referred to as tDNA. Any T nucleotide of the tRNA in encoded form in the synthetic DNA construct will be replaced with a U nucleotide when the encoded tRNA is transcribed. The synthetic DNA construct comprises or consists of a DNA encoding a tRNA structurally modified in that the tRNA, i.e., the tRNA resulting from transcription of the synthetic construct, has a structurally modified D arm, anticodon arm, variable loop and/or T arm. The synthetic DNA construct may include regulatory elements, e.g., 5' leader sequences comprising promoters and the like, other than those described above for the synthetic DNA construct according to the first aspect of the invention, affecting, for example, transcription. The DNA construct may be a synthetic vector, e.g., expression vector for the delivery and expression of the tRNA in a cell, e.g., human cell.

The anticodon arm of the tRNA encoded by the construct, for example may have one of the following general structures with a modified stem portion (see above):
5'-GCAGG-AC-Loop-CCTGT-3'
5'-TTGGG-AC-Loop-CTCAA-3'
5'-TTGGA-AC-Loop-TTCAA-3'
5'-ATGGT-AC-Loop-ACCAT-3'
5'-GCGGA-AC-Loop-TCCGC-3'
5'-GCGGT-AC-Loop-ACCGC-3'
5'-GGCGG-AC-Loop-CCGCC-3'
5'-GGCGC-AC-Loop-GCGCC-3'
5'-TTGGG-AC-loop-CCCAA-3'
5'-CTGGA-AC-loop-TCCAG-3'
5'-CCGGA-AC-loop-TCCGG-3'
5'-GCTGC-AC-loop-GCAGT-3'

Preferred anticodon arms of the encoded tRNA may have one of the sequences of SEQ ID NO: 52-115 (see above):
In further preferred embodiments of the invention, the encoded tRNA comprises, in combination with one of the AC arms mentioned above, or alone, i.e. not in combination with one of the AC arms mentioned above, a variable loop (V loop) having one of the following sequences of SEQ ID NO: 116-117 (see above).

In a further preferred embodiment of the invention, the encoded tRNA of the invention comprises, in combination with one of the AC arms described above and/or in combination with one of the V loops described above, or alone, i.e. not in combination with one of the AC arms mentioned above and/or not in combination with one of the V loops mentioned above, a T arm having the following general sequence (see above):
GCGGG-T-Loop-CCCGT
ACGGG-T-Loop-CCCGT
GTAGG-T-Loop-CCCAT
GTCGG-T-Loop-CCCGT
GGCGG-T-Loop-CCGGT
GCAGG-T-Loop-CCCGT
GCCGG-T-Loop-CCGGT

Preferably, the T-Loop (positions 54-60 according to tRNA numbering convention) has the sequence HTCGANT, H being A, C, or T, i.e., CTCGANT, ATCGANT or TTCGANT, further preferred TTCGANT, for example TTCGAAT or TTCGAGT, preferably TTCGAGT.

In preferred embodiments, the encoded tRNA of the invention comprises, for example, a T arm of one of the sequences of SEQ ID NO: 118-126.

In a further preferred embodiment of the invention, the tRNA encoded in the synthetic DNA construct according to this aspect of the invention comprises, in combination with one of the AC arms described above and/or in combination with one of the V loops described above and/or in combination with one of the T arms described above, or alone, i.e. not in combination with one of the AC arms mentioned above and/or not in combination with one of the V loops mentioned above and/or not in combination with one of the T arms described above, an acceptor stem having the following general sequence:
5'-GGCTCTG-rest tRNA-CAGAGTC-3'
5'-GGCGCTG-rest tRNA-CAGCGTC-3'
5'-GGCGCGG-rest tRNA-CGGCGTC-3'

The term "rest tRNA" refers to the part of the tRNA between the 5' and 3' portions of the acceptor stem, and includes the D arm, V loops and T arm. Of course, the "rest tRNA" is not to be considered a part of the acceptor stem.

In a preferred embodiment, the synthetic DNA construct of the invention consists of or comprises a nucleic acid encoding a transfer RNA, the encoded transfer RNA comprising
a) an anticodon arm having or comprising one of the sequences of SEQ ID NO: 52-115, and/or
b) a variable loop having or comprising one of the sequences of SEQ ID NO: 116-117, and/or
c) a T arm having or comprising one of the sequences according to SEQ ID NO: 118-126, and/or
d) an acceptor stem having the structure 5'-GGCTCTG-rest tRNA-CAGAGTC-3' or 5'-GGCGCTG-rest tRNA-CAGCGTC-3'.

The "rest tRNA" is not to be considered a part of the acceptor stem.

It should be noted that a reference to a tRNA structure (e.g., T arm, AC arm etc.) within the DNA construct is to be understood as a reference to the DNA sequence(s) encoding the structure in the DNA construct. When transcribed into a mature tRNA, the sequences will be transcribed into RNA sequences forming the structure in the mature tRNA.

In a further aspect the invention relates to a pharmaceutical composition comprising the synthetic DNA construct according to the invention, as described immediately above this paragraph and a pharmaceutically acceptable carrier. An example of a simple carrier is a buffer solution or a physiologic salt solution.

The invention will be described in the following by way of examples and the appended figures for illustrative purposes only.
Figure 1. Schematic drawing of a generalized "consensus" tRNA structure and its numbering according to tRNA numbering convention.
Figure 2. Schematic drawing of part of three embodiments of the first embodiment of a synthetic DNA construct of the invention.
Figure 3. Rescue activity of various suppressor tRNA variants.

Figure 1 depicts an example of a tRNA numbered according to the conventional numbering applied to a generalized "consensus" tRNA, beginning with 1 at the 5' end and ending with 76 at the 3' end. The tRNA is composed of tRNA nucleotides 11 and has the common cloverleaf structure of tRNA comprising an acceptor stem 2 with the CCA tail 10, a T arm 3 with the TψC loop 6, a D arm 4 with the D loop 7 and an anticodon arm 5 with a five-nucleotide stem portion 8 and the anticodon loop 9. In such a "consensus" tRNA the nucleotides of the natural anticodon triplet 25 is always at positions 34, 35 and 36, regardless of the actual number of previous nucleotides. A tRNA may, for example, contain additional nucleotides between positions 1 and 34, e.g. in the D loop, and in the variable loop 24 between positions 45 and 46. Additional nucleotides may be numbered with added alphabetic characters, e.g. 20a, 20b etc. In the variable loop 24 the additional nucleotides are numbered with a preceding "e" and a following numeral depending on the position of the nucleotide in the loop. Positions of modified nucleotides within a tRNA of the invention are marked as black filled circles.

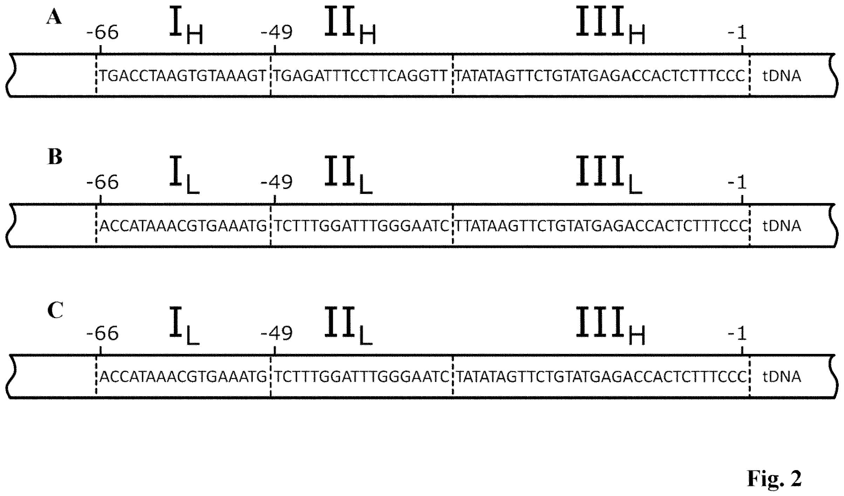

Figure 2 schematically shows part of three embodiments of a first embodiment of a synthetic DNA construct of the invention including the sequence motifs IL, IL, IIH, IIL, IIIH and IIIL in three different combinations. Fig. 2A shows an embodiment of a DNA construct including the sequence motifs I_{H}, II_{H} and III_{H} (P1, SEQ ID NO: 12), Fig 2B shows an embodiment of a vector including the sequence motifs I_{L}, II_{L} and III_{L} (P2, SEQ ID NO: 13), and Fig. 2C shows an embodiment of a vector including the sequence motifs I_{L}, II_{L} and III_{H} (P23, SEQ ID NO: 34).

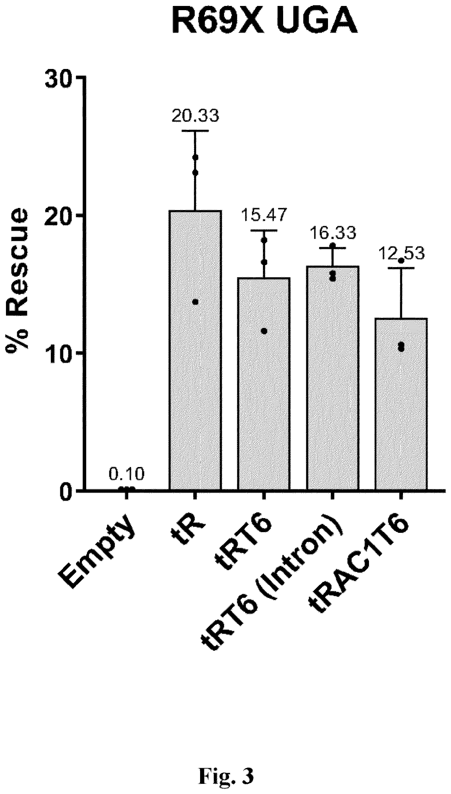

Figure 3 shows the rescue activity of various suppressor tRNA variants. tRNA variants embedded in a separate vector were co-transfected with a vector encoding firefly luciferase with a PTC (TGA) in place of an arginine codon (CGA, "R69X UGA") in HEK293 cells. The luciferase expression was measured 24h post-transfection and the tRNA suppression activity (% rescue) is presented as percentage of the wild-type luciferase. The following suppressor tRNAs were considered: (i) tR, (ii) tRT6, (iii) tRT6 retaining an intron sequence and (iv) TRAC1T6. Mock transfected cells (empty) served as negative control. For this, cells were transfected with an empty vector without luciferase, but subjected to the same transfection procedure, i.e. treated with same amounts of lipofectamine which alters slightly cell growth.

To test readthrough efficiency of suppressor tRNA a reporter plasmid was used with Firefly luciferase (FLuc) driven by EF1a, to achieve the desired arginine PTC, the 69th codon of FLuc in the reporter which is arginine was mutated to a stop codon UGA, embedded in pTwist EF1 Alpha Puro plasmid backbone. The tRNA was also encoded using a plasmid system in which the desired suppressor tRNA as its DNA form, some of them retaining the intron, driven by U6 promoter embedded in pcDNA3.1 plasmid backbone.

HEK293 cells were seeded in 96-well cell culture plates at 1×10⁴ cells/well and grown in Dulbecco's Modified Essential Medium (DMEM, Pan Biotech) supplemented with 10% fetal bovine serum (FBS, Pan Biotech) and 2 mM L-glutamine (Thermo Fisher Scientific). 16 to 24 hours later, cells were co-transfected in triplicate with 25 ng R69X PTC-FLuc or WT Flue plasmids and 100 ng of each suppressor tRNA variant or empty control plasmids using lipofectamine 3000 (Thermo Fisher Scientific). After four to six hours, medium was replaced and 24 hours post-transfection cells were lysed with 1x passive lysis buffer (Promega) and luciferase activity measured with luciferase assay system (Promega) and Spark microplate reader (Tecan). Readthrough activity was presented as percentage of the activity of WT luciferase that was expressed on a separate expression vector.

First, the anticodon of tRNA^{Arg} (TCT 3-1, source tRNA data base: http://gtrnadb.ucsc.edu/genomes/eukaryota/Hsapi38/) was exchanged to pair to UGA PTC, thus creating tR (Fig. 3). We next changed the TψC stem (creating tRT5 variant) and simultaneously TψC stem and acceptor stem (tRAC1T6 variant) in tR. In addition, the natural intron of tRNA^{Arg} was kept (variant tRT6 (intron)), as introns usually provide an expression benefit to tRNAs. For the activity screen, all tRNA variants were cloned into the plasmid (pTwist EF 1 Alpha Puro) and co-transfected in HEK293 cells together with a plasmid bearing the firefly luciferase (FLuc) reporter with arginine PTC (R69X). After 24h, the luciferase activity was measured and normalized to that of the wildtype luciferase (% rescue, Fig. 3). All tRNA variants efficiently restored the expression of R69X luc in a range of 12-20% (of the wildtype luciferase; numbers displayed in Fig. 3 (n=3 independent biological replicates) are mean values of restored luciferase activity).

The sequences of the tRNA mentioned above are as follows (anticodon underlined; T stem, AC stem, T stem and acceptor stem double underlined; intron in lower case letters):
tR (based on native tRNA-Arg-TCT-3-1 (Arg-chr9.tRNA5), replaced UCA anticodon, 5'-3 ; SEQ ID NO: 127): 
GGCTCTGTGGCGCAATGGATAGCGCATTGGACTTCAAATTCAAAGGTTGTGGGTTC
GAGTCCCACCAGAGTCG
tRT6 (Arg-chr9.tRNA5 with modified T-stem, 5'-3'; SEQ ID NO: 128): 
GGCTCTGTGGCGCAATGGATAGCGCATTGGACTTCAAATTCAAAGGTTGCGGGTTC
GAGTCCCGTCAGAGTCG
tRT6 (Intron) (Arg-chr9.tRNA5 maintaining the intron and with modified T-stem, 5'-3'; SEQ ID NO: 129): 
GGCTCTGTGGCGCAATGGATAGCGCATTGGACTTCAAgctgagcctagtgtggtcATTCAAAG
GTTGCGGGTTCGAGTCCCGTCAGAGTCG
tRAc1T6 (tRT6 with changes in the acceptor stem; SEQ ID NO: 130):

GGCGCTGTGGCGCAATGGATAGCGCATTGGACUTCAAATTCAAAGGTTGCGGGTTC
GAGTCCCGTCAGCGTCG

The positions of the D stem, anticodon stem, T stem and acceptor stem in the above tRNA are as follows (anticodon 34..36):
Sequences without intron (SEQ ID NO: 127, 128, 130)

| | 5' part | 3' part |
|---|---|---|
| Acceptor stem: | 1..7; | 66..72 |
| D stem: | 10..13; | 22..25 |
| Anticodon stem: | 27..31; | 39..43 |
| T stem: | 49..53 | 61..65 |

Sequence with intron (SEQ ID NO: 129; intron: 38..55)

| | 5' part | 3' part |
|---|---|---|
| Acceptor stem: | 1..7; | 84..90 |
| D stem: | 10..13; | 22..25 |
| Anticodon stem: | 27..31; | 57..61 |
| T stem: | 67..71 | 79..83 |

## Claims

1. A synthetic DNA construct comprising (A) a nucleic acid encoding a transfer RNA and (B) a 5' leader sequence, the 5' leader sequence comprising
a) at least one sequence motif selected from the group consisting of the sequence motifs TGACCTAAGTGTAAAGT, I_{H}, (SEQ ID NO: 1), TGAGATTTCCTTCAGGTT, II_{H}, (SEQ ID NO: 2), TATATAGTTCTGTATGAGACCACTCTTTCCC, III_{H}, (SEQ ID NO: 3), ACCATAAACGTGAAATG, I_{L}, (SEQ ID NO: 4), TCTTTGGATTTGGGAATC, II_{L}, (SEQ ID NO: 5, and TTATAAGTTCTGTATGAGACCACTCTTTCCC, III_{L}, (SEQ ID NO: 6); or
b) one sequence motif selected from the group consisting of the sequence motifs VNANANTVHANANNTTNNNATRANATTCNGDGVGNAANATABTNCTVGND, 50nt_{H}, (SEQ ID NO: 7) and
GCNGGDGGCGNGTTCBBCTGTNVNAGCNTNCGDNGNCGTTTNNCNTCGGT, 50nt_{L}, (SEQ ID NO: 8); or
c) at least one sequence motif selected from the group consisting of the sequence motifs GAAATGCCTT, 10nt_{H1}, (SEQ ID NO: 9), GTGGGAACTA 10nt_{H2}, (SEQ ID NO: 10), and GTGTTGCTTG 10nt_{H3}, (SEQ ID NO: 11).

2. The synthetic DNA construct according to claim 1, wherein,
aa) in relation to a) above,
i. the 5' leader sequence does not comprise the sequence motif I_{H} if it comprises the sequence motif I_{L}, and vice versa,
ii. the 5' leader sequence does not comprise the sequence motif II_{H} if it comprises the sequence motif II_{L}, and vice versa, and
iii. the 5' leader sequence does not comprise the sequence motif III_{H} if it comprises the sequence motif III_{L}, and vice versa; or
bb) in relation to b) above, the 5' leader sequence does not comprise the sequence motif 50nt_{H} if it comprises the sequence motif 50nt_{L}, and vice versa.

3. The synthetic DNA construct according to one of claims 1 or 2, wherein,
aa) in relation to a) above,
i. if the 5' leader sequence comprises the sequence motif I_{H} or I_{L}, the sequence motif I_{H} or I_{L} has, in 5'-3' direction, the positions -66 to -50,
ii. if the 5' leader sequence comprises the sequence motif II_{H} or II_{L}, the sequence motif II_{H} or II_{L} has, in 5'-3' direction, the positions -49 to -32, and
iii. if the 5' leader sequence comprises the sequence motif III_{H} or III_{L}, the sequence motif III_{H} or III_{L} has, in 5'-3' direction, the positions -31 to -1; or
bb) in relation to b) above, the sequence motif 50nt_{H}, or 50nt_{L} has, in 5'-3' direction, the positions -50 to -1.

4. The synthetic DNA construct according to one of claims 1 to 3, wherein the 5' leader sequence comprises,
aa) in relation to a) above, at least two sequence motifs selected from the sequence motifs I_{L}, II_{L}, III_{L}, I_{H}, II_{H} or III_{H}, and wherein the at least two sequence motifs are arranged, in 5'-3' direction, in the order I_{H} - II_{H}, II_{H} - III_{H}, I_{H} - III_{H}, I_{L} - II_{L}, II_{L} - III_{L}, I_{L} - III_{L}, I_{H} - II_{L}, I_{H} - III_{L}, II_{H} - III_{L}, I_{L} - II_{H}, II_{L} - III_{H}, or I_{L} - III_{H}; or
cc) in relation to c) above, at least two sequence motifs of the sequences motifs 10nt_{H1}, 10nt_{H2}, and 10nt_{H3}, the at least two sequence motifs being:
i. GAAATGCCTT, 10nt_{H1}, (SEQ ID NO: 9) and GTGTTGCTTG, 10nt_{H3}, (SEQ ID NO:11), wherein the sequence motifs are arranged, in 5'-3' direction, in the following order:
10nt_{H3} - 10nt_{H1}, or
ii. GAAATGCCTT, 10nt_{H1}, (SEQ ID NO: 9) and GTGGGAACTA, 10nt_{H2}, (SEQ ID NO:10), wherein the sequence motifs are arranged, in 5'-3' direction, in the following order:
10nt_{H1} - 10nt_{H2}, or
iii. GTGGGAACTA, 10nt_{H2}, (SEQ ID NO:10), and GTGTTGCTTG, 10nt_{H3}, (SEQ ID NO:11), wherein the sequence motifs are arranged, in 5'-3' direction, in the following order: 10nt_{H3} - 10nt_{H2}.

5. The synthetic DNA construct according to any of the preceding claims, wherein the 5' leader sequence comprises
aa) in relation to a) above, three sequence motifs selected from the sequence motifs I_{L}, II_{L}, III_{L}, I_{H}, II_{H} or III_{H}, preferably in the following order, in 5'-3' direction: I_{L} - II_{L} - III_{L}, I_{H} - II_{H} - III_{H}, I_{H} - II_{L} - III_{L}, I_{L} - II_{H} - III_{L}, I_{L} - II_{L} - III_{H}, I_{H} - II_{H} - III_{L}, I_{H} - II_{L} - III_{H}, or I_{L} - II_{H} - III_{H}; or
cc) in relation to c) above all three sequence motifs 10nt_{H1}, 10nt_{H2}, and 10nt_{H3}, preferably in the following order, in 5'-3' direction: 10nt_{H3} - 10nt_{H1} - 10nt_{H2}.

6. The synthetic DNA construct according to any of the preceding claims, wherein the 5' leader sequence has or comprises
aa) in relation to a) above, a sequence according to one of SEQ ID NO: 12-37
bb) in relation to b) above, a sequence according to one of SEQ ID NO: 38-51.

7. The synthetic DNA construct according to any of the preceding claims, further comprising the coding sequence for a natural or synthetic transfer RNA comprising a B box comprising the sequence H₅₄T₅₅C₅₆G₅₇A₅₈N₅₉T₆₀, preferably T₅₄T₅₅C₅₆G₅₇A₅₈N₅₉T₆₀, the index representing the positions of the nucleotides in the transfer RNA, the position numbering following transfer RNA numbering convention.

8. The synthetic DNA construct according to any of the preceding claims, the tRNA encoded by the nucleic acid comprising:
a) an anticodon arm having or comprising one of the sequences of SEQ ID NO: 52-115, and/or
b) a variable loop having or comprising one of the sequences of SEQ ID NO: 116-117, and/or
c) a T arm having or comprising one of the sequences according to SEQ ID NO: 118-126, and/or
d) an acceptor stem having the structure 5'-GGCUCUG-rest tRNA-CAGAGUC-3' or 5'-GGCGCUG-rest tRNA-CAGCGUC-3'.

9. The synthetic DNA construct according to any of the preceding claims, wherein the synthetic DNA construct is a synthetic vector, preferably a viral vector.

10. A pharmaceutical composition comprising the synthetic DNA construct according to one of the preceding claims and a pharmaceutically acceptable carrier.

11. The synthetic DNA construct according to one of claims 1 to 9 or the pharmaceutical composition according to claim 10 for use as a medicament.

12. The synthetic DNA construct according to one of claims 1 to 9 or the pharmaceutical composition according to claim 10 for use as a medicament in a disease, which is at least partly caused by a nonsense or frameshift mutation leading to the production of a protein being dysfunctional or non-functional compared to the wild-type protein, or at least partly caused by the intracellular sequestration of a tRNA leading to depletion of the cellular pool of the tRNA.

13. The synthetic DNA construct according to one of claims 1 to 9 or the pharmaceutical composition according to claim 10 for use as a medicament for treating Hurler syndrome, beta-thalassemia, Crohn's disease, Tay-Sachs disease, Duchenne muscular dystrophy, cystic fibrosis, neuronal ceroid lipofuscinosis, neurofibromatosis type 1, or Charcot-Marie-Tooth disease.

14. A synthetic DNA construct consisting of or comprising a nucleic acid encoding a transfer RNA, the encoded transfer RNA comprising
a) an anticodon arm having or comprising one of the sequences of SEQ ID NO: 52-115, and/or
b) a variable loop having or comprising one of the sequences of SEQ ID NO: 116-117, and/or
c) a T arm having or comprising one of the sequences according to SEQ ID NO: 118-126, and/or
d) an acceptor stem having the structure 5'-GGCTCTG-rest tRNA-CAGAGTC-3' or 5'-GGCGCTG-rest tRNA-CAGCGTC-3'.
